(19) Europäisches Patentamt

European Patent Office

Office européen des brevets

(11) **EP 1 695 721 A1**

(12) **EUROPÄISCHE PATENTANMELDUNG**

(43) Veröffentlichungstag:
  **30.08.2006 Patentblatt 2006/35**

(51) Int Cl.:
  *A61L 15/26* *(2006.01)*    *A61L 15/58* *(2006.01)*

(21) Anmeldenummer: **06007469.7**

(22) Anmeldetag: **20.09.2001**

(84) Benannte Vertragsstaaten:
  **AT BE CH CY DE DK ES FI FR GB GR IE IT LI LU MC NL PT SE TR**
  Benannte Erstreckungsstaaten:
  **AL LT LV MK RO SI**

(30) Priorität: **22.09.2000 DE 10047884**

(62) Dokumentnummer(n) der früheren Anmeldung(en) nach Art. 76 EPÜ:
  **01122413.6 / 1 190 723**

(71) Anmelder: **Beiersdorf Aktiengesellschaft D-20253 Hamburg (DE)**

(72) Erfinder:
  • **Kenndoff, Jochen, Dr. 21075 Hamburg (DE)**
  • **Schink, Michael, Dr. 22609 Hamburg (DE)**
  • **Leutz, Reiner 21465 Reinbek (DE)**
  • **Sachau, Günther 25451 Quickborn (DE)**
  • **Ahrens, Helge 22297 Hamburg (DE)**

Bemerkungen:
  Diese Anmeldung ist am 08 - 04 - 2006 als Teilanmeldung zu der unter INID-Kode 62 erwähnten Anmeldung eingereicht worden.

(54) **Selbsthaftende Wundverbände mit haftfähigem Wundversorgungsbereich**

(57)    Die Erfindung betrifft einen selbsthaftenden Wundverband mit einem haftfähigen Wundversorgungsbereich, der insbesondere an Druckstellen, an Blasen und offenen Blasen an der Ferse, am Handballen und an den Fingern eingesetzt werden kann.

**Figur 2**

EP 1 695 721 A1

**Beschreibung**

Die Erfindung betrifft einen selbsthaftenden Wundverband mit einem haftfähigen Wundversorgungsbereich, der insbesondere an Druckstellen, an Blasen und offenen Blasen an der Ferse, am Handballen und an den Fingern eingesetzt werden kann.

Moderne Wundversorgungsprodukte wie Hydrokolloide (siehe hierzu zum Beispiel "Hydrokolloide" von R. Lipmann in "Medical Device & Diagnostic Industry, June 1999), die für Kolostomie-und professionelle Wundversorgungsanwendungen entwickelt wurden, finden zunehmend Anwendung.

Wundversorgungsprodukte auf Basis von Hydrokolloiden weisen Vorteile gegenüber herkömmlichen Pflastern auf. Diese generieren ein feuchtes Wundheilungsmilieu, das die Wunde nicht austrocknen lässt und ein optimales Milieu zur schnellen Wundheilung erzeugt. Weitere Vorteile sind die Unauffälligkeit bei der Anwendung, sicheres Haftvermögen, Absorptionsvermögen von Exudat (zum Beispiel aus einer Blase stammend), gute Polsterwirkung und die schmerzlose Entfernbarkeit.

Typische Zusammensetzungen der ersten kommerziell erhältlichen Hydrokolloiden für Wundversorgungsprodukte umfassen:

- Niedermolulares Polyisobutylen (40 Gew.-%),
- Pektin (20 Gew.-%),
- Natriumcarboxymethylcellulose = CMC (20 Gew.-%)
- Gelatine (20 Gew.-%)

Moderne Hydrokolloid-Formulierungen, so genannte integrierte Formulierungen, wie sie zum Beispiel von der Firma Coloplast angeboten werden, basieren auf Styrol-Isopren-Styrol-Blockpolymeren mit Kohlenwasserstoffharzen ("hydrocarbon resins") als Tackifiern, Mineralöl als Weichmacher sowie CMC als Absorber.

Diese Formulierungen mit SIS als Gerüstbildner enthalten glasartige Domänen (Styrol-Blöcke) und thermoplastische Domänen (Isopren-Blöcke). Bei Raumtemperatur sorgen die glasartigen Domänen für eine Art dreidimensionale "cross-linking"-Struktur, die bei höherer Temperatur aufgehoben wird.

Hydrokolloide zeigen auch bei feuchten Bedingungen einen guten "wet-tack", so dass sie für die Anwendung als Blasenpflaster an Ferse und Handinnenfläche, einem Applikationsort mit relativ hohem Feuchtigkeitsverlust der Haut, gut geeignet sind.

Aus der EP 0 264 299 B1 ist ein Verband bekannt, der aus einem wasserabsorbierenden Abdichtungskissen besteht, das seinerseits von einem oder mehreren Hydrokolloiden gebildet wird. Das oder die Hydrokolloide sind in einem Bindemittel aufgelöst oder mit demselben gemischt.

Das Kissen ist fest von einer wasserdichten Deckungsschicht vollständig umfaßt. Erfindungsgemäß ist das Kissen mindestens um die äußere Peripherie auf eine solche Weise abgeschrägt, dass die Dicke am Rand etwa ein Viertel ihrer maximalen Dicke nicht überschreitet.

Die Herstellung erfolgt über ein Druckgussverfahren bei hohen Drücken und hohen Temperaturen. Dieses Verfahren ist für vernetzte Polymergele beispielsweise Polyurethangele nicht geeignet.

Konturierte Wundauflagen mit einer Klebmasseschicht bestehend aus quellbaren Hydrokolloiden und wasserunlöslichen, viskosen Bestandteilen, beispielsweise Polyisobutylen, Kautschuk, Silicon oder Polyurethanelastomeren, sind Gegenstand der WO 92/05755.

Dabei hat die Klebmasseschicht im Randbereich, die von derselben Art ist wie die Klebmasse im zentralen Bereich, eine Dicke von kleiner 0,5 mm (bevorzugt kleiner 0,3 mm) und eine Breite von mindestens 5 mm (bevorzugt mindestens 10 mm). Die Klebmasse auf Basis von Hydrokolloid zeigt auch auf feuchtem Grund einen Tack.

Wasserfreie Hydrogele werden als Xerogele bezeichnet und sind makromolekulare, natürliche oder synthetische Stoffe, die aufgrund eines hohen Gehaltes an hydrophilen Gruppen in der Lage sind, Wasser absorbtiv zu binden. Die Wasseraufnahmekapazität vieler Xerogele beträgt das Mehrfache des Eigengewichtes der wasserfreien Substanz.

Hydrogele oder Xerogele werden in vielfältiger Form in der Wundversorgung eingesetzt, denn sie schützen Wunden vor der Austrocknung, saugen Wundsekret auf, dienen als Matrix für Wirkstoffe aller Art und auch als Basis für die Besiedelung mit autologen oder heterologen Hautzellen.

Gele können unter anderem in Form von Schäumen verwendet werden. Schäume zur Versorgung von Hautwunden oder chirurgischen Wunden sind dem Fachmann an sich bekannt. Hauptsächlich finden dabei Polyurethanschäume oder Kollagenschäume Verwendung.

Auch selbstklebende Gelschäume sind dem Fachmann bekannt. Diese lassen sich zwar im allgemeinen recht gut auf der Haut fixieren, haben aber meistens den Nachteil, dass ihre Wasseraufnahmekapazität und ihr Wasserrückhaltevermögen stark eingeschränkt sind.

Weiterhin sind hydrophile Schäume aus Polyurethangelen bekannt. Die WO 88/01878 A1 beschreibt selbstklebende Polyurethanschäume beziehungsweise Polyurethanschaumgele, welche unter anderem einpolymerisierte Methacrylate enthalten können. Die Herstellung dieser Schaumgele erfolgt durch Zusatz von Wasser.

Polyurethangele auf der Basis einer Polyurethanmatrix und höhermolekularen Polyolen werden auch in EP 0 057 839 B1 beschrieben. Selbsthaftende Flächengebilde aus Polyurethangelen sind aus EP 0 147 588 B1 bekannt. Die in diesen beiden letztgenannten Schriften offenbarten Polyurethangele sind ungeschäumt.

Die selbstklebenden Gele haben Isocyanatkennzahlen von 15 bis 70 (EP 0 147 588 A2).

EP 0 196 364 A2 beschreibt hydrophile Polyurethanschäume, die mit wasserabsorbierenden Polymeren auf Basis eines Copolymers der Acrylsäure und Kaliumacetat gefüllt sein können und für medizinische Zwecke gedacht sind. Das Polyurethan wird auf Basis von MDI hergestellt. Der eingesetzte Polyether hat eine Mindestfunktionalität von zwei Hydroxylgruppen, bevorzugt je zwei bis drei Hydroxylgruppen. Das Verhältnis NCO/OH ist stöchiometrisch. Damit handelt es sich nicht um ein gelförmiges Polyurethan. Geschäumt werden kann mit Druckluft oder mit anderen, nicht mit dem Isocyanat reagierenden Gasen oder mit Hilfe von niedrig siedenden Lösungsmitteln. Die Mischung von Absorber mit Polyetherpolyol erfolgt etwa im Verhältnis 3:1.

Der Schaum hat klebende Eigenschaften auf Wunden, die durch ein aluminisiertes Vlies gänzlich unterdrückt werden müssen, um ihn zur Wundbehandlung einsetzen zu können.

Schaumwundauflagen, wie sie zum Beispiel von der Firma Beiersdorf unter dem Namen Cutinova ® thin und Cutinova ® hydro erhältlich sind, sind u. a. beschrieben in DE 42 33 289 A1, in DE 196 18 825 A1 und WO 97/43328.

Danach besteht der Polyurethangelschaum aus einem Polyadditionsprodukt eines Polyetherpolyols (Levagel ® von Bayer AG) mit einem aromatischen oder aliphatischen Diisocyanat (Desmodur ® Bayer AG), in das ein Polyacrylat-Superabsorber-Pulver (Favor ® , Stockhausen) eingearbeitet wurde. Das Polyurethangel kann, je nach Verhältnis von OH-Äquivalenten des Polyols zu reaktiven Isocyanat-Gruppen, schwach oder stark selbsthaftend auf Haut eingestellt werden.

Der flächige Polyurethangelschaum mit einer Dicke von 1 bis 6 mm wird einseitig von einer Polyurethanfolie abgedeckt. Pflaster entsprechender Größe werden aus der Ballenware ausgestanzt. Die so hergestellte Wundauflage entklebt überraschenderweise vollständig bei Aufnahme von Wundflüssigkeit und zeigt dabei nicht die von Hydrokolloiden bekannte Neigung zur Desintegration bei starker Quellung, die dazu führen kann, dass Rückstände des Hydrokolloids in der Wunde verbleiben.

Die ausgestanzten großflächigen Wundauflagen eignen sich hervorragend zur Versorgung von chronischen oder schwerheilenden Wunden von Patienten, die stationär versorgt werden müssen.

Bei kleineren Bagatell-Verletzungen oder Blasenbildung aufgrund von Druckstellen an Hand, Ballen und Ferse zeigt dieser Produktaufbau jedoch einige Nachteile.

Das Produkt rollt sich aufgrund seiner Stanzränder bei mechanischer Belastung leicht auf. Bei Kontakt mit Feuchtigkeit erweisen sich die offenen Stanzränder als Nachteil, da dadurch Wasser an die saugende Schicht gelangen kann und zur Aufquellung und Entklebung des Polyurethangels durch seitliches Eindringen von Feuchtigkeit führt.

Weiterhin wird durch die signifikante Produkthöhe (bis zu 4 mm) und den gleichen selbstklebenden Eigenschaften am Rand das Anhaften von Schmutz und ein Aufrollen durch Anhaften von zum Beispiel Kleidungsstücken begünstigt.

Ein Verfahren zur Herstellung eines Verbandes mit dünnen Rändern bestehend aus mindestens zwei Klebmasseschichten wird in EP 0 680 299 A1 beschrieben. Die Klebstoffschichten, die von derselben oder von unterschiedlicher Art sein können, entsprechen einer Anordnung von miteinander verbundenen unterschiedlichen Flächen, die in ihrer Größe zur Spitze abnehmen. Dabei weisen die einzelnen Schichten ein Stufenprofil auf, welches, um einen nach außen kontinuierlichen Verlauf zu erzielen, mit einer weiteren Schicht abgedeckt werden muss. Weiterhin nachteilhaft ist die stufenweise Abflachung des Verbandes auf der wundzugewandten Seite, so dass dadurch an einigen Stellen im Wund- und Wundrandbereich der Kontakt ungleichmäßig erfolgt.

In EP 0 919 211 A2 wird die Herstellung von Wundverbänden mit abgeschrägten Rändern aus thermogeformten Kunststoffträgerfilmen, die trennbeschichtet sind und eine Kavität aufweisen, in die ein selbstklebendes, hydrophiles Polymergel gebracht wird, genannt. Die Verbände weisen eine klebende Deckschicht auf, die wiederum mit einer Schutzschicht abgedeckt ist. Das Verfahren ist aufwendig und für Wundverbände aus "einem Guss" nicht geeignet. Auch hier sind die Verbände auf der wundzugewandten Seite abgeschrägt.

Schließlich können noch herkömmliche Pflaster zur Versorgung von Wunden eingesetzt werden (zum Beispiel das Gewebepflaster Hansaplast® classic der Firma Beiersdorf), die sich nur bedingt für die Anwendung als Blasenpflaster zur Versorgung von Druckstellen beziehungsweise geschädigter Hornhaut an stark konturierten Körperpartien eignen.

Nachteilig erweisen sich die geringe Elastizität und die Neigung zum Aufrollen des Trägermaterials an den Kanten des Pflasters bei mechanischer Belastung bei längerer Tragedauer. Zusätzlich wird das Pflaster bei täglicher Körperpflege oder beim Händewaschen stark durchfeuchtet und verliert an Haftvermögen.

Herkömmliche Pflaster fallen optisch stark auf, behindern Bewegungen beziehungsweise beeinträchtigen das Tragegefühl im Schuh.

Aufgabe der Erfindung ist es, einen Wundverband zur Verfügung zu stellen, welcher in der Lage ist, Exudat aus Wunden aufzusaugen, welcher gut polstert, welcher ausreichend Feuchtigkeit von der Haut durch das Pflaster nach außen transportiert und welcher ein feuchtes Wundheilungsmilieu erzeugt.

Gelöst wird die Aufgabe durch einen Wundverband, wie er im Anspruch 1 dargelegt ist. Die Unteransprüche umfassen vorteilhafte Varianten des Erfindungsgegenstands.

Demgemäß betritt die Erfindung einen Wundverband aus einer wasserdampfdurchlässigen Polyurethan-Matrix, die sich ausgehend von einem Punkt des Wundverbands, insbesondere mittig gelegen, zum Rand hin abschrägt, wobei die wasserdampfdurchlässige Polyurethan-Matrix vollflächig auf einer Trägerfolie aufgetragen ist.

Insbesondere liegt der Punkt im Flächenzentrum, um ein symmetrisches Aussehen des Pflasters zu erzielen. Die Abschrägung kann aber auch unregelmäßig erfolgen, je nach Bedarf und Anwendungsfall des Pflasters.

Es ergeben sich auf diese Weise unterschiedlichste Formen. Die Matrix kann zum Beispiel linsenförmig geformt oder halbkugelförmig sein.

Die Höhe der Randzone beträgt maximal 50% von der Gesamthöhe des Produktes, bevorzugt werden Höhen der Randzone von kleiner 0,2 mm. Der Konturierungsverlauf vom Zentrum zum Rand wird durch die gewählte Form festgelegt, das heißt, die Gießform bestimmt das Design der Kontur.

Weiter vorzugsweise besteht die insbesondere flexible, wasserdampfdurchlässige oder okklusive Trägerfolie aus Polyurethan, Polyethylen, Polypropylen, Polyamid oder Polyester und/oder weist eine Dicke zwischen 60 bis 80 $\mu$m auf. Diese Aufzählung ist aber nicht abschließend zu verstehen, selbstverständlich kann der Fachmann ohne erfinderisches Zutun andere, geeignete Folien auffinden.

Gegebenenfalls wird das selbstklebende Produkt durch ein silikonisiertes Papier oder eine insbesondere silikonisierte Folie abgedeckt, so dass die klebende Seite während der Lagerung geschützt ist.

Weiter vorzugsweise sind in der Polyurethan-Matrix ein oder mehrere Wirkstoffe enthalten.

Typische Wirkstoffe sind - ohne den Anspruch der Vollständigkeit im Rahmen der vorliegenden Erfindung zu erheben:

| Indikation: | Wirkstoff |
| --- | --- |
| Antimykotika | Nafitin |
| | Amorrolfin |
| | Tolnaftat |
| | Ciclopirox |
| Antiseptika | Thymol |
| | Eugenol |
| | Triclosan |
| | Hexachlorophen |
| | Benzalkoniumchlorid |
| | Clioquinol |
| | Chinolinol |
| | Undecensäure |
| | Ethacridin |
| | Chlorhexidin |
| | Hexetidin |
| | Dodicin |
| | Iod |
| Nichtsteroidale Antirheumatika | Glykolsalicylat |
| | Flufenaminsäure |
| | Etofenamat |
| | Ketoprofen |
| | Piroxicam |
| | Indomethacin |
| Antipuriginosa | Polidocanol |
| | Isoprenalin |
| | Crotamiton |
| Lokalanästhetika | Benzocain |
| Antipsoriatika | Ammoniumbitumasulfonat |

| Indikation: | Wirkstoff |
|---|---|
| Keratolytika | Harnstoff |

Die Polyurethanmasse kann ungeschäumt, geschäumt, ungefüllt oder mit zusätzlichen Füllstoffen, wie beispielsweise Superabsorbern, Titandioxid, Zinkoxid, Weichmachern, Farbstoffen etc. eingesetzt werden. Für Anwendungen im Bereich transdermaler Pflastersysteme ist es auch möglich, die Polyurethanmasse in der zentralen Zone mit Wirkstoffen zu dotieren. Weiterhin können auch Hydrogele in halbfester bis fester Form mit aktiven Bestandteilen für die zentrale Zone verwendet werden.

Geeignete Polyurethane für die Matrix sind Gegenstand der DE 196 18 825, in der hydrophile, selbstklebende Polyurethan-Gele offenbart werden, die bestehen aus

a) 2 bis 6 Hydroxylgruppen aufweisenden Polyetherpolyolen mit OH-Zahlen von 20 bis 112 und einem Ethylenoxid (EO)-Gehalt von ≥ 10 Gew.-%,
b) Antioxidantien,
c) in den Polyolen a) löslichen Wismut-(HI)-Carboxylaten auf Basis von Carbonsäuren mit 2 bis 18 C-Atomen als Katalysatoren sowie
d) Hexamethylendiisocyanat,

mit einem Produkt der Funktionalitäten der Polyurethan-bildenden Komponenten a) und d) von mindestens 5,2, wobei die Katalysatormenge c) 0,005 bis 0,25 Gew.-%, bezogen auf das Polyol a) beträgt, die Menge an Antioxidantien b) im Bereich von 0,1 bis 1,0 Gew.-%, bezogen auf Polyol a) liegt und ein Verhältnis von freien NCO-Gruppen der Komponente d) zu den freien OH-Gruppen der Komponente a) (Isocyanatkennzahl) im Bereich von 0,30 bis 0,70 gewählt wird.

Es werden bevorzugt 3 bis 4, ganz besonders bevorzugt 4-Hydroxylgruppen aufweisende Polyetherpolyole eingesetzt mit einer OH-Zahl im Bereich von 20 bis 112, bevorzugt 30 bis 56. Der Ethylenoxidgehalt liegt bei den erfindungsgemäß eingesetzten Polyetherpolyolen bei vorzugsweise ≥ 20 Gew.-%.

Die Polyetherpolyole sind als solche an sich bekannt und werden zum Beispiel durch Polymerisation von Epoxiden, wie Ethylenoxid, Propylenoxid, Butylenoxid oder Tetrahydrofuran, mit sich selbst oder durch Anlagerung dieser Epoxide, vorzugsweise von Ethylenoxid und Propylenoxid - gegebenenfalls im Gemisch untereinander oder separat nacheinander - an Starterkomponenten mit mindestens zwei reaktionsfähigen Wasserstoffatomen, wie Wasser, Ethylenglykol, Propylenglykol, Diethylenglykol, Dipropylenglykol, Glyzerin, Trimethylolpropan, Pentaerythrit, Sorbit oder Succrose, hergestellt. Vertreter der genannten, zu verwendenden höhermolekularen Polyhydroxylverbindungen sind zum Beispiel in High Polymers, Vol. XVI, "Polyurethanes, Chemistry and Technology" (Saunders-Frisch, Interscience Publishers, New York, Bd 1, 1962, S. 32-42) aufgeführt.

Als Isocyanatkomponente wird monomeres oder trimerisiertes Hexamethylendiisocyanat oder durch Biuret-, Uretdion-, Allophanatgruppen oder durch Prepolymerisierung mit Polyetherpolyolen oder Mischungen von Polyetherpolyolen auf Basis der bekannten Starterkomponenten mit 2 oder > 2 reaktionsfähigen H-Atomen und Epoxiden, wie Ethylenoxid oder Propylenoxid einer OH-Zahl von ≤ 850, bevorzugt 100 bis 600, modifiziertes Hexamethylendiisocyanat eingesetzt. Bevorzugt ist der Einsatz von modifiziertem Hexamethylendiisocyanat, insbesondere durch Prepolymerisierung mit Polyetherdiolen der OH-Zahl 200 bis 600 modifiziertes Hexamethylen-diisocyanat. Ganz besonders bevorzugt sind Modifizierungen des Hexamethylendiisocyanats mit Polyetherdiolen der OH-Zahl 200-600, deren Restgehalt an monomeren Hexamethylendiisocyanat unter 0,5 Gew.-% liegt.

Als Katalysatoren kommen für die erfindungsgemäßen Polyurethangele in den wasserfreien Polyetherpolyolen a) lösliche Wismut(III)-Carboxylate auf Basis linearer, verzweigter, gesättigter oder ungesättigter Carbonsäuren mit 2 bis 18, vorzugsweise 6 bis 18 C-Atomen in Frage. Bevorzugt sind Bi(III)Salze verzweigter gesättigter Carbonsäuren mit tertiären Carboxylgruppen, wie der 2,2-Dimethyl- Octansäure (zum Beispiel Versatic-Säuren, Shell). Gut geeignet sind Zubereitungen dieser Bi (III) Salze in überschüssigen Anteilen dieser Carbonsäuren. Hervorragend bewährt hat sich eine Lösung von 1 mol des Bi(III)Salzes der Versatic 10-Säure (2,2-Dimethyloctansäure) in einem Überschuß von 3 mol dieser Säure mit einem Bi-Gehalt von ca. 17%.

Es werden die Katalysatoren bevorzugt in Mengen von 0,03 bis 0,1 Gew.-%, bezogen auf das Polyol a), eingesetzt.

Als Antioxidantien kommen für die erfindungsgemäßen Polyurethan-Gele insbesondere sterisch gehinderte phenolische Stabilisatoren, wie BHT (2,6-Di-tert.butyl-4-methylphenol), Vulkanox BKF (2,2 min -Methylen-bis-(6-tert.-butyl-4-methyl phenol) (Bayer AG), Irganox 1010 (Pentaerythrityl-tetrakis-[3-(3,5-ditert.-butyl-4- hydroxyphenyl)-propionat]), lrganox 1076 (Octadecyl-3-(3,5-ditert.-butyl-4- hydroxyphenyl)-propionat) (Ciba-Geigy) oder Tocopherol (Vitamin E) in Betracht. Bevorzugt werden solche vom Typ des α-Tocopherol eingesetzt.

Die Antioxidantien werden bevorzugt in Mengen von 0,15 bis 0,5 Gew.-%, bezogen auf das Polyol a), eingesetzt.

Die Isocyanatkennzahl (Verhältnis der bei der Reaktion eingesetzten freien NCO-Gruppen zu den freien OH-Gruppen) der erfindungsgemäßen Polyurethan-Gelmassen liegt je nach der Funktionalität der eingesetzten Isocyanat- und Polyolkomponenten im Bereich von 0,30 bis 0,70, bevorzugt im Bereich von 0,45 bis 0,60. Die für eine Gelbildung erforderliche Isocyanatkennzahl kann sehr einfach nach der folgenden Formel abgeschätzt werden:

$$f_{(Polyol)} \bullet (f_{(Isocyanat)} - 1) \bullet Kennzahl \approx 2$$

$$Kennzahl \approx \frac{2}{f_{(Polyol)} \bullet (f_{(Isocyanat)} - 1)}$$

f: Funktionalität der Isocyanat- oder Polyolkomponente

Je nach angestrebter Klebrigkeit oder Elastizität des Gels kann die tatsächlich zu verwendende Isocyanatkennzahl um bis zu + 20% von dem berechneten Wert abweichen.

Die erfindungsgemäßen Polyurethan-Gelmassen werden hergestellt nach üblichen Verfahren, wie sie beispielsweise beschrieben sind in Becker/Braun, Kunststoff- Handbuch, Bd. 7, Polyurethane, S. 121 ff, Carl-Hauser, 1983.

Weiter vorzugsweise kommen Polyurethane zum Einsatz, wie sie in der EP 0 665 856 B1 offenbart sind.

Die hydrophilen Polyurethangelschäume sind demnach erhältlich aus

1. einem Polyurethangel, welches

(A) 25-62 Gew.-%, vorzugsweise 30-60 Gew.-%, besonders bevorzugt 40-57 Gew.-%, bezogen auf die Summe aus (A) und (B), eines kovalent vernetzten Polyurethans als hochmolekulare Matrix und

(B) 75-38 Gew.-%, vorzugsweise 70-40 Gew.-%, besonders bevorzugt 60-43 Gew.-%, bezogen auf die Summe aus (A) und (B) einer oder mehrerer in der Matrix durch Nebenvalenzkräfte fest gebundenen Polyhydroxylverbindungen mit einem mittleren Molekulargewicht zwischen 1000 und 12000, vorzugsweise zwischen 1500 und 8000, besonders bevorzugt zwischen 2000 und 6000, und einer mittleren OH-Zahl zwischen 20 und 112, vorzugsweise zwischen 25 und 84, besonders bevorzugt zwischen 28 und 56, als flüssigem Dispersionsmittel, wobei das Dispersionsmittel im wesentlichen frei ist an Hydroxylverbindungen mit einem Molekulargewicht unter 800, vorzugsweise unter 1000, besonders bevorzugt unter 1500, sowie gegebenenfalls

(C) 0 bis 100 Gew.-%, bezogen auf die Summe aus (A) und (B), an Füll- und/oder Zusatzstoffen enthält,

a) und welches erhältlich ist durch Umsetzung einer Mischung von

b) einem oder mehreren Polyisocyanaten,

c) einer oder mehreren Polyhydroxylverbindungen mit einem mittleren Molekulargewicht zwischen 1000 und 12000, und einer mittleren OH-Zahl zwischen 20 und 112,

d) gegebenenfalls Katalysatoren oder Beschleunigern für die Reaktion zwischen Isocyanat-und Hydroxylgruppen sowie gegebenenfalls

e) aus der Polyurethanchemie an sich bekannten Füll- und Zusatzstoffen,

wobei diese Mischung im wesentlichen frei ist von Hydroxylverbindungen mit einem Molekulargewicht unter 800, die mittlere Funktionalität der Polyisocyanate ($F_I$) zwischen 2 und 4 liegt, die mittlere Funktionalität der Polyhydroxylverbindung (Fp) zwischen 3 und 6 beträgt und die Isocyanatkennzahl (K) der Formel

$$K = \frac{300 \pm X}{(F_l \bullet F_p) - 1} + 7$$

gehorcht, in welcher $X \leq 120$, vorzugsweise $X \leq 100$, besonders bevorzugt $X \leq 90$ ist und die Kennzahl K bei Werten zwischen 15 und 70 liegt, wobei die angegebenen Mittelwerte von Molekulargewicht und OH-Zahl als Zahlenmittel zu verstehen sind,

2. einem Wasser absorbierenden Material und

3. einem nichtwässrigen Schäumungsmittel.

Die Polyurethangele können aus den an sich aus der Polyurethanchemie bekannten Ausgangsverbindungen nach an sich bekannten Verfahren hergestellt werden, wie sie zum Beispiel in DE 31 03 499 A1, DE 31 03 500 A1 und EP 0 147 588 A1 beschrieben werden. Wesentlich ist jedoch, dass bei der Auswahl der gelbildenden Komponenten die oben definierten Bedingungen eingehalten werden, da sonst anstelle von selbsthaftenden Gelen klebfreie, elastische Gele erhalten werden.

Bevorzugte Polyhydroxylverbindungen sind Polyetherpolyole, wie sie in den oben genannten Offenlegungsschriften ausführlich genannt sind.

Als Polyisocyanatkomponenten sind sowohl (cyclo)aliphatische als auch aromatische Isocyanate geeignet. Bevorzugte (cyclo)aliphatische Polyisocyanate sind 1,6-Hexamethylen-diisocyanat sowie dessen Biurete und Trimerisate beziehungsweise hydrierte Diphenylmethandiisocyanat ("MDI")-Typen. Bevorzugte aromatischen Polyisocyanate sind solche, die durch Destillation erhalten werden, wie MDI-Gemische aus 4,4'- und 2,4'-Isomeren oder 4,4'-MDI, sowie Toluylendiisocyanat ("TDI")-Typen.

Die Diisocyanate können insbesondere zum Beispiel aus der Gruppe der unmodifizierten aromatischen oder aliphatischen Diisocyanate oder aber aus durch Prepolymerisierung mit Aminen, Polyolen oder Polyetherpolyolen gebildeten modifizierten Produkten gewählt werden.

Die Polyurethan-Gele können gegebenenfalls aus der Polyurethan-Chemie an sich bekannte Zusatzstoffe enthalten, wie zum Beispiel Füllstoffe und Kurzfasern auf anorganischer oder organischer Basis, Metallpigmente, oberflächenaktive Substanzen oder flüssige Streckmittel wie Substanzen mit einem Siedepunkt von über 150 °C.

Als organische Füllstoffe seien beispielsweise Schwerspat, Kreide, Gips, Kieserit, Soda, Titandioxid, Ceroxid, Quarzsand, Kaolin, Russ und Mikrohohlkugeln genannt.

An organischen Füllstoffen können zum Beispiel Pulver auf Basis von Polystyrol, Polyvinylchlorid, Harnstoff-Formaldehyd und Polyhydrazodicarbonamid eingesetzt werden. Als Kurzfasern kommen zum Beispiel Glasfasern von 0,1 - 1 mm Länge oder Fasern organischer Herkunft, wie zum Beispiel Polyester- oder Polyamidfasern, in Frage. Metallpulver, wie zum Beispiel Eisen oder Kupferpulver, können ebenfalls bei der Gelbildung mitverwendet werden. Um den Gelen die gewünschte Färbung zu verleihen, können die bei der Einfärbung von Polyurethanen an sich bekannten Farbstoffe oder Farbpigmente auf organischer oder anorganischer Basis verwendet werden, wie zum Beispiel Eisenoxidoder Chromoxidpigmente, Pigmente auf Phthalocyanin- oder Monoazo-Basis. Als oberflächenaktive Substanzen seien zum Beispiel Cellulosepulver, Aktivkohle und Kieselsäurepräparate genannt.

Zur Modifizierung der Hafteigenschaften der Gele können gegebenenfalls Zusätze von polymeren Vinylverbindungen, Polyacrylaten und sonstigen in der Klebstoff-Technik üblichen Copolymeren beziehungsweise auch Klebemittel auf Naturstoffbasis bis zu einem Gehalt von 10 Gew.-%, bezogen auf das Gewicht der Gelmasse, zugegeben werden.

Bevorzugte Wasser absorbierende Materialien sind als Superabsorber bekannte Wasser absorbierende Salze von Polyacrylaten und deren Copolymeren, insbesondere die Natrium- oder Kaliumsalze. Sie können unvernetzt oder vernetzt sein und sind auch als Handelsprodukte erhältlich. Insbesondere sind solche Produkte geeignet, wie sie in der DE 37 13 601 A1 offenbart werden und auch Superabsorber der neuen Generation mit nur noch geringen Anteilen an austrockenbarem Wasser und hohem Quellvermögen unter Druck.

Bevorzugte Produkte sind schwach vernetzte Polymerisate auf der Basis Acrylsäure/Natriumacrylat. Solche Natrium-polyacrylate sind als Favor (Chemische Fabrik Stockhausen GmbH, Deutschland) erhältlich.

Weitere Absorber, zum Beispiel Carboxymethylcellulose und Karaya, sind ebenfalls geeignet.

Der Schäumungsgrad lässt sich durch die eingearbeiteten Mengen an Schäumungsmittel in weiten Grenzen variieren.

Im Randbereich nicht saugende, haftende Wundverbände weisen durch ihren Produktaufbau eine vorteilhafte Kombination von guter Produktverklebung auf der Haut auf, die das Produkt während der Anwendung vor Aufrollen sowie Eindringen von Feuchtigkeit schützt und gutes Absorptionsvermögen von Wundsekret bei gleichzeitiger Entklebung im zentralen Bereich des Wundversorgungsproduktes gewährleistet. Die Funktionalität des Produktes kann somit gegenüber herkömmlichen Produkten deutlich verlängert werden und wird außerdem durch äußere Einwirkung von Feuchtigkeit (Baden, Duschen) nicht beeinträchtigt.

Die Herstellung derartig geschützter selbstklebender und saugender erfindungsgemäßer Wundauflagenbereiche erfolgt durch Dosieren und Aufbringen einer definierten Menge eines noch flüssigen Polyurethangels oder Polyurethanschaumes in eine Form, die mit einem flexiblen Trägermaterial (mit selbstklebenden, nicht-klebenden oder trennenden Eigenschaften) ausgelegt ist. Nach Aufbringen des Polyurethans erfolgt das Abdecken der äußeren Schicht mit einem Abdeckmaterial (ebenfalls mit selbstklebenden, nicht-klebenden oder trennenden Eigenschaften in Abhängigkeit vom Bestimmungszweck und dem eingesetzten Trägermaterial) und die anschließende Aushärtung des Polyurethans zu einem festen Gel oder Schaum.

Es ist auch möglich, die flüssige Polyurethanmasse zunächst auf ein trennendes Abdeckmaterial zu bringen und anschließend noch vor der Verfestigung durch Aufbringen einer Form, die mit einem flexiblen Trägermaterial (mit selbstklebenden, nicht-klebenden oder trennenden Eigenschaften) ausgelegt ist, die Verformung des Polyurethangels oder Polyurethanschaumes durchzuführen.

Durch beide Herstellverfahren wird die erfindungsgemäße Herstellung von ein- oder mehrschichtigen, naturgemäß dreidimensional konturierten Wundversorgungsprodukten ermöglicht. Der Wundauflagenbereich kann dabei einer herkömmlichen Form entsprechen (eine auf das Trägermaterial aufgebrachte erhabene zum Beispiel rechteckige Wundauflage oder beispielsweise linsenförmig gekrümmt sein mit nach außen konvexer Form oder kombinierten konvexen und konkaven Elementen, wobei die wundzugewandte Fläche plan ist.

Die Geometrien der herstellbaren konturierten Formkörper sind vielfältig (rund, elliptisch, quadratisch, dreieckig etc.). Die klebende Fläche besteht im einfachsten Fall aus einer Polyurethanklebmasse (Gel oder Schaum) "in einem Guss".

Die Verarbeitungszeit der reaktiven Polyurethanmasse wird über die Topfzeit, die zwischen 2 und 10 Minuten liegen sollte, kontrolliert.

Die beschriebenen Verfahren heben sich gegenüber herkömmlichen Verfahren besonders durch die Materialeinsparung infolge der Dosierung und Verarbeitung definierter Mengen ab.

Im folgenden sollen anhand von Bildern sowie eines Beispiele besonders vorteilhafte Ausführungsformen des Verbands dargestellt werden.

**Beispiele**

Herstellung von PU-Formkörper mit flachen Rändern

**Vergleichsbeispiel 1**

1-Schichtaufbau

Material:

| | |
|---|---|
| Gießform: | 6 cm Durchmesser; 1,8 mm tief |
| Stanzform: | 10 cm Durchmesser |
| 9 g PU-Masse | mit einer Isocyanatkennzahl (KZ) von 0,47 auf einer Polyisocyanat (HDI-Basis)/Polyetherpolyol enthaltend |
| | 10 Gew.-% Superabsorber Favor T |
| | 0,4 Gew.-% Vitamin E |
| | 0,08 Gew.-% Coscat 83 (Katalysator auf Bi-Basis) |

Trennfolie (silikonisierte PE-Folie) und Trennpapier

Durchführung:

Trennfolie in die Form einlegen, Masse zudosieren, mit Trennpapier abdecken, mit planer Platte belegen und 2 kg Gewicht belasten, nach Aushärtung stanzen.

<pre>
Muster:   PU-Matrix;
          Dicke in der Mitte 2,3 mm;
          Dicke am Rand 0,7 mm
</pre>

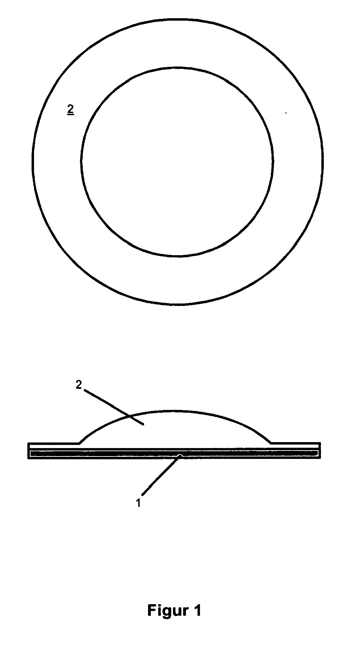

Die Figur 1 verdeutlicht eine geometrische Form des Wundverbands, wie er insbesondere für Blasenpflaster zum Einsatz kommt.

Das Pflaster weist eine kreisrunde Form (Durchmesser 100 mm) auf, besteht aus einer wasserdampfdurchlässigen Polyurethan-Matrix (2), die sich zum Rand hin abschrägt. Die Polyurethan-Matrix (2) schrägt sich zunächst gleichmäßig ab und läuft in einem 20 mm breiten Ring aus, bei dem die Dicke konstant beibehalten wird. Die Polyurethan-Matrix (2) ist mittig im wesentlichen halbkonvex ausgeformt, ist demgemäß einer halbkonvexen Linse vergleichbar.

Die Dicke der Polyurethan-Matrix 2 beträgt in der Mitte 2,3 mm und am Rande 0,7 mm.

Schließlich ist die Polyurethan-Matrix (2) mit einem silikonisierten Papier (1) eingedeckt, um eine Verschmutzung oder Kontamination der Matrix zu vermeiden.

**Beispiel 2**

2-Schichtaufbau

<div align="center">Material:</div>

| | |
|---|---|
| Gießform: | oval (25 x46 mm); 1,3 mm tief |
| Stanzform: | oval (42 x 68 mm) |
| 2 g PU-Masse | mit einer Isocyanatkennzahl (KZ) von 0,47 auf einer Polyisocyanat (HDI-Basis)/Polyetherpolyol enthaltend |
| | 10 Gew.-% Superabsorber Favor T |
| | 0,4 Gew.-% Vitamin E |
| | 0,08 Gew.-% Coscat 83 (Katalysator auf Bi-Basis) |

Trennfolie (silikonisierte PE-Folie) und Trennpapier

Durchführung:

PE-Folie in die Form einlegen, Masse zudosieren, mit Trennpapier abdecken, mit planer Platte belegen und 2 kg Gewicht belasten, nach Aushärtung stanzen.

<pre>
Muster:   Abdeckung PE-Folie
          PU-Masse als Wundabdeckung und Randverklebung;
          Dicke in der Mitte 1,6 mm;
          Dicke am Rand 0,3 mm
</pre>

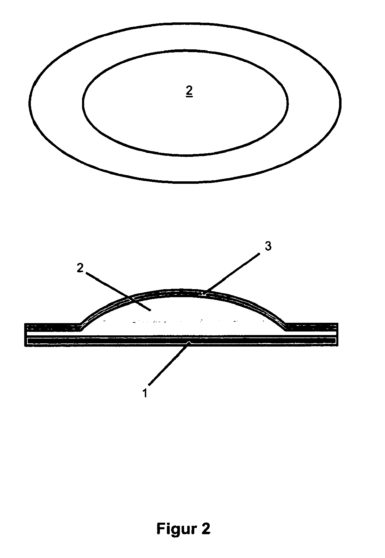

Die Figur 2 verdeutlicht eine bevorzugte geometrische Form des Wundverbands.

Das Pflaster weist eine ellipsoide Form (Länge der Achsen 42 mm beziehungsweise 68 mm) auf, besteht aus einer wasserdampfdurchlässigen Polyurethan-Matrix 2, die sich zum Rand hin abschrägt. Die Polyurethan-Matrix 2 schrägt sich zunächst gleichmäßig ab und läuft in einem ungefähr 11 mm breiten Ring aus, bei dem die Dicke konstant beibehalten wird. Die Polyurethan-Matrix 2 ist mittig im wesentlichen halbkonvex ausgeformt, ist demgemäß einer halbkonvexen Linse vergleichbar.

Die PU-Matrix 2 ist auf der hautabgewandten Seite mit einer PE-Folie 3 eingedeckt. Die Dicke der Polyurethan-Matrix 2 samt PE-Folie 3 beträgt in der Mitte 1,6 mm und am Rande 0,3 mm.

Schließlich ist die Polyurethan-Matrix 2 mit einem silikonisierten Papier eingedeckt, um eine Verschmutzung oder Kontamination der Matrix 2 zu vermeiden.

**Vergleichsbeispiele 3.1. bis 3.3**

3- Schichtaufbau

Beispiel 3.1.

Material:

| | |
|---|---|
| Gießform: | oval (34 x72mm); 1,2 mm tief |
| Stanzform: | oval (65 x 110 mm) |
| 1,2 g PU-Masse | mit einer Isocyanatkennzahl (KZ) von 0,48 auf einer Polyisocyanat (HDI-Basis)/Polyetherpolyol enthaltend |
| | 22,5 Gew.-% Superabsorber Favor T |

PU-Folie klebend ausgerüstet mit PU-Masse, mit 20 % Isopropylpalmitat (IPP) gefüllt; Trennpapier

Durchführung:

PE-Folie in die Form einlegen, Masse zudosieren, mit Trennpapier abdecken, mit planer Platte belegen und 2 kg Gewicht belasten, nach Aushärtung stanzen.

| | |
|---|---|
| Muster: | Abdeckung PE-Folie; |
| | PU-Masse mit Superabsorber als Wundabdeckung und PU-Masse mit IPP als Randverklebung |
| | Dicke in der Mitte 1,3 mm; |
| | Dicke am Rand 0,15 mm |

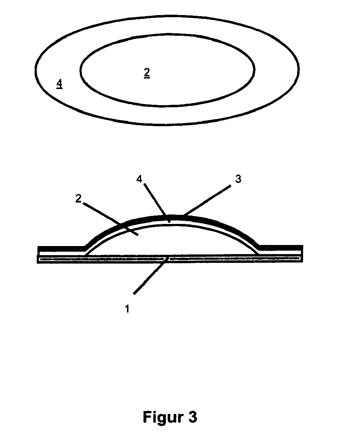

Die Figur 3 verdeutlicht eine weitere bevorzugte geometrische Form des Wundverbands.

Das Pflaster weist eine eilipsoide Form auf (Länge der Achsen 110 mm beziehungsweise 65 mm), besteht aus einer wasserdampfdurchlässigen Polyurethan-Matrix 2, die sich zum Rand hin abschrägt. Die Polyurethan-Matrix 2 ist im wesentlichen halbkonvex ausgeformt, ist demgemäß einer halbkonvexen Linse mit einer Länge der Achsen von 72 mm beziehungsweise 34 mm vergleichbar.

Die PU-Matrix 2 ist auf der hautabgewandten Seite mit einer PE-Folie 3 eingedeckt, die mit der Klebeschicht 4 auf Polyurethan-Basis, die IPP enthält, vollflächig beschichtet ist. In der hier gezeigten Ausführungsform des Pflasters ist die gesamte Peripherie der Klebeschicht 4 nicht mit der Polyurethan-Matrix 2 bedeckt. Es ergeben sich auf diese Weise zwei konzentrische Zonen chemisch unterschiedlicher Klebemassen 2, 4, welche sich hinsichtlich Haftvermögen, Absorptionsvermögen und Polstereigenschaft unterscheiden.

Die Dicke der Polyurethan-Matrix 2 samt PU-Folie 3 und Klebeschicht 4 beträgt in der Mitte 1,3 mm und am Rande 0,15 mm.

Schließlich ist die Polyurethan-Matrix 2 mit einem silikonisierten Papier eingedeckt, um eine Verschmutzung oder Kontamination der Matrix 2 zu vermeiden.

Vergleichsbeispiel 3.2

Material:

| | |
|---|---|
| Gießform: | 6 cm Durchmesser; 1,8 mm tief |
| Stanzform: | 10 cm Durchmesser |
| | 1,5 g PU-Masse geschäumt, Dichte 0,65 g/cm$^3$ |
| | mit einer Isocyanatkennzahl (KZ) von 0,46 auf einer Polyisocyanat (HDI-Basis)/Polyetherpolyol enthaltend |
| | 22,5 Gew.-% Superabsorber Favor T |

PU-Folie klebend ausgerüstet mit Acrylatmasse, Trennpapier

<u>Durchführung:</u>

PU-Folie in die Form einlegen, Masse zudosieren, mit Trennpapier abdecken, mit planer Platte belegen und 2 kg Gewicht belasten, nach Aushärtung stanzen.

> Muster: Abdeckung PU -Folie;
> PU-Masse, geschäumt als Wundabdeckung und Acrylatmasse als Rand-verklebung
> Dicke in der Mitte 1,5 mm;
> Dicke am Rand 0,10 mm

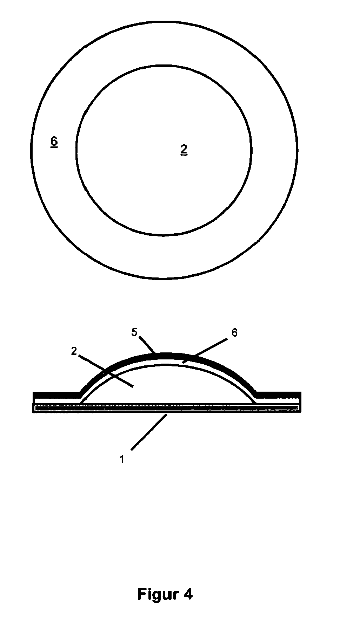

Die Figur 4 verdeutlicht eine weitere bevorzugte geometrische Form des Wundverbands.

Das Pflaster weist eine kreisrunde Form auf (Durchmesser 100 mm), besteht aus einer wasserdampfdurchlässigen geschäumten Polyurethan-Matrix 2, die sich zum Rand hin abschrägt. Die Polyurethan-Matrix 2 ist im wesentlichen halbkonvex ausgeformt, ist demgemäß einer halbkonvexen Linse mit einem Durchmesser von 60 mm vergleichbar.

Die PU-Matrix 2 ist auf der hautabgewandten Seite mit einer PU-Folie 3 eingedeckt, die mit der Klebeschicht 6 auf Acrylat-Basis vollflächig beschichtet ist. In der hier gezeigten Ausführungsform des Pflasters ist die gesamte Peripherie der Klebeschicht 6 nicht mit der Polyurethan-Matrix 2 bedeckt. Es ergeben sich auf diese Weise zwei konzentrische Zonen chemisch unterschiedlicher Klebemassen 2, 6, welche sich hinsichtlich Haftvermögen, Absorptionsvermögen und Polstereigenschaft unterscheiden.

Die Dicke der Polyurethan-Matrix 2 samt PU-Folie 3 und Klebeschicht 6 beträgt in der Mitte 1,5 mm und am Rande 0,1 mm.

Schließlich ist die Polyurethan-Matrix 2 mit einem silikonisierten Papier eingedeckt, um eine Verschmutzung oder Kontamination der Matrix 2 zu vermeiden.

**Vergleichsbeispiel 3.3**

> Material:
> Gießform: 3,3 cm Durchmesser; 1,5 mm tief
> Stanzform: 5 x 5 cm, Ecken gerundet
> 0,75 g PU-Masse mit einer Isocyanatkennzahl (KZ) von 0,47 auf einer Polyisocyanat (HDI-Basis)/Polyetherpolyol enthaltend
> 10 Gew.-% Superabsorber Favor T

PE-Folie, hautfarben,
klebend ausgerüstet mit Kautschukmasse;
Trennpapier

<u>Durchführung:</u>

PE-Folie in die Form einlegen, Masse zudosieren, mit Trennpapier abdecken, mit planer Platte belegen und 2 kg Gewicht belasten, nach Aushärtung stanzen.

> Muster: Abdeckung PE-Folie;
> PU-Masse als Wundabdeckung und Kautschukmasse als Randverkle-bung
> Dicke in der Mitte 1,5 mm;
> Dicke am Rand 0,10 mm

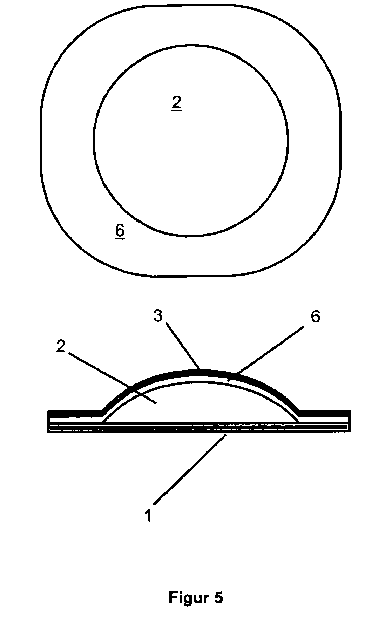

Die Figur 5 verdeutlicht eine weitere bevorzugte geometrische Form des Wundverbands.

Das Pflaster weist eine quadratische Form auf, wobei die Ecken des Quadrats abgerundet sind (Durchmesser des Quadrats 50 mm), besteht aus einer wasserdampfdurchlässigen geschäumten Polyurethan-Matrix 2, die sich zum Rand hin abschrägt. Die Polyurethan-Matrix 2 ist im wesentlichen halbkonvex ausgeformt und kreisrund, ist demgemäß einer halbkonvexen Linse mit einem Durchmesser von 33 mm vergleichbar.

Die PU-Matrix 2 ist auf der hautabgewandten Seite mit einer PU-Folie 3 eingedeckt, die mit der Klebeschicht 6 auf Kautschuk-Basis vollflächig beschichtet ist. In der hier gezeigten Ausführungsform des Pflasters ist die gesamte Peripherie der Klebeschicht 6 nicht mit der Polyurethan-Matrix 2 bedeckt. Es ergeben sich auf diese Weise zwei konzentrische Zonen chemisch unterschiedlicher Klebemassen 2, 6, welche sich hinsichtlich Haftvermögen, Absorptionsvermögen und Polstereigenschaft unterscheiden.

Die Dicke der Polyurethan-Matrix 2 samt PU-Folie 3 und Klebeschicht 6 beträgt in der Mitte 1,5 mm und am Rande 0,1 mm.

Schließlich ist die Polyurethan-Matrix 2 mit einem silikonisierten Papier eingedeckt, um eine Verschmutzung oder Kontamination der Matrix 2 zu vermeiden.

**Patentansprüche**

1.   Wundverband, umfassend eine wasserdampfdurchlässige selbstklebende Polyurethan-Matrix, die sich ausgehend von einem Punkt des Wundverbands, insbesondere mittig gelegen, zum Rand hin abschrägt, **dadurch gekennzeichnet, dass** die wasserdampfdurchlässige Polyurethan-Matrix vollflächig auf eine Trägerfolie aufgetragen ist.

2.   Wundverband nach den Anspruch 2, **dadurch gekennzeichnet, dass** die Trägerfolie aus Polyurethan, Polyethylen, Polypropylen, Polyamid oder Polyester besteht.

3.   Wundverband nach den Ansprüchen 2 oder 3, **dadurch gekennzeichnet, dass** die Trägerfolie eine Dicke zwischen 60 bis 80 $\mu$m aufweist.

4.   Wundverband nach zumindest einem der vorherigen Ansprüche, **dadurch gekennzeichnet, dass** der Wundverband über seine ganze Breite bis zum Gebrauch mit einem klebstoffabweisenden Trägermaterial, wie silikonisiertes Papier, abgedeckt ist.

5.   Wundverband nach zumindest einem der vorherigen Ansprüche, **dadurch gekennzeichnet, dass** in der Polyurethan-Matrix ein oder mehrere Wirkstoffe enthalten sind.

**Figur 1**

**Figur 2**

**Figur 3**

**Figur 4**

**Figur 5**

**Europäisches Patentamt**

# EUROPÄISCHER RECHERCHENBERICHT

Nummer der Anmeldung

EP 06 00 7469

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| D,X | EP 0 147 588 A (BAYER AG) 10. Juli 1985 (1985-07-10) * Seite 3, Zeile 29 * * Seite 11, Zeile 14 - Seite 12, Zeile 8 * * Seite 13, Zeile 17 - Zeile 21 * * Seite 14, Zeile 8 - Zeile 10 * * Beispiele 3,9 * ----- | 1-5 | INV. A61L15/26 A61L15/58 |
| E | EP 1 190 722 A (BEIERSDORF AG) 27. März 2002 (2002-03-27) * das ganze Dokument * ----- | 1-5 | |
| D,A | WO 92/05755 A (JENSEN OLE R) 16. April 1992 (1992-04-16) * Seite 5, letzter Absatz - Seite 7, letzter Absatz * * Seite 11, Absatz 2 - Seite 13, Absatz 3 * * Abbildung 8 * ----- | 1-5 | |
| A | EP 0 919 211 A (BEIERSDORF AG) 2. Juni 1999 (1999-06-02) * Seite 2, Spalte 1, Zeile 27 - Zeile 39 * * Seite 3, Spalte 3, Zeile 36 - Spalte 4, Zeile 6 * * Abbildungen 1,2 * ----- | 1-5 | RECHERCHIERTE SACHGEBIETE (IPC) A61L |
| D,A | EP 0 264 299 A (COLOPLAST AS) 20. April 1988 (1988-04-20) * Zusammenfassung * ----- | 1-5 | |

Der vorliegende Recherchenbericht wurde für alle Patentansprüche erstellt

| Recherchenort | Abschlußdatum der Recherche | Prüfer |
|---|---|---|
| München | 19. Juli 2006 | Hars, J |

EPO FORM 1503 03.82 (P04C03)

## ANHANG ZUM EUROPÄISCHEN RECHERCHENBERICHT
## ÜBER DIE EUROPÄISCHE PATENTANMELDUNG NR.

EP 06 00 7469

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

19-07-2006

| Im Recherchenbericht angeführtes Patentdokument | Datum der Veröffentlichung | Mitglied(er) der Patentfamilie | Datum der Veröffentlichung |
|---|---|---|---|
| EP 0147588 A | 10-07-1985 | AU 570744 B2 | 24-03-1988 |
| | | AU 3546684 A | 23-05-1985 |
| | | CA 1250190 A1 | 21-02-1989 |
| | | DE 3341555 A1 | 30-05-1985 |
| | | ES 8603929 A1 | 16-05-1986 |
| | | GR 80947 A1 | 26-02-1985 |
| | | IL 73514 A | 31-10-1989 |
| | | JP 1622491 C | 25-10-1991 |
| | | JP 2042394 B | 21-09-1990 |
| | | JP 60118783 A | 26-06-1985 |
| | | KR 9310440 B1 | 25-10-1993 |
| | | PT 79501 A | 01-12-1984 |
| | | US 4661099 A | 28-04-1987 |
| EP 1190722 A | 27-03-2002 | AT 323515 T | 15-05-2006 |
| | | AU 6997001 A | 28-03-2002 |
| | | DE 10047673 A1 | 11-04-2002 |
| | | US 2002156411 A1 | 24-10-2002 |
| WO 9205755 A | 16-04-1992 | DE 69124687 D1 | 27-03-1997 |
| | | DE 69124687 T2 | 31-07-1997 |
| | | DK 503029 T3 | 10-03-1997 |
| | | EP 0503029 A1 | 16-09-1992 |
| | | ES 2097217 T3 | 01-04-1997 |
| | | JP 2572919 B2 | 16-01-1997 |
| | | JP 5503871 T | 24-06-1993 |
| EP 0919211 A | 02-06-1999 | KEINE | |
| EP 0264299 A | 20-04-1988 | DE 3783427 D1 | 18-02-1993 |
| | | DE 3783427 T2 | 30-05-1996 |
| | | DK 497686 A | 18-04-1988 |
| | | ES 2037726 T3 | 01-07-1993 |
| | | US 4867748 A | 19-09-1989 |

EPO FORM P0461

Für nähere Einzelheiten zu diesem Anhang : siehe Amtsblatt des Europäischen Patentamts, Nr.12/82